# EUROPÄISCHE PATENTANMELDUNG

(11) **EP 0 595 004 A1**
(43) Veröffentlichungstag der Anmeldung: **04.05.1994**
(21) Anmeldenummer: 93114731.8
(22) Anmeldetag: 14.09.1993
(51) Int. Cl.: A61L 2/20

(54) **Verfahren zur Sterilisierung und Rückgewinnung eines Sterilisiergases**

(30) Priorität: 29.10.1992 DE 4236622
(71) Anmelder: HERCO KÜHLTECHNIK HERMANNS & CO. GmbH, D-46474 Wesel (DE); AIR PRODUCTS GmbH, D-45523 Hattingen (DE)
(72) Erfinder: Karthaus, Michael, D-41469 Neuss (DE); Hermanns, Klaus, Dr., D-46569 Hünxe (DE)
(74) Vertreter: Schwabe - Sandmair - Marx

(57) **Zusammenfassung**

Bei einem Verfahren zur Sterilisierung mit Ethylenoxid (EO) und Rückgewinnung des EO werden die zu sterilisierenden Materialien in eine Sterilisierkammer verbracht und die Kammer zunächst auf auf einen Druck von 100 mbar oder weniger evakuiert. Die Kammer wird anschließend mit einem Gasgemisch aus EO und Stickstoff soweit bedruckt, daß die in der Kammer verbliebene Restluft weniger als etwa 10-Vol.% der Kammeratmosphäre ausmacht. Nach Einwirkung des Gasgemisches wird die Kammer unter Abzug des Gasgemisches wieder auf einen Druck von etwa 100 mbar oder weniger evakuiert und aus dem abgezogenen Gasgemisch das EO in einem Tieftemperaturkondensator zurückgewonnen, danach wird sie mindestens einmal mit Stickstoff wieder bedruckt und anschließend wieder auf einen Druck von etwa 100 mbar oder weniger evakuiert.

## Beschreibung

Die Erfindung betrifft ein Verfahren zur Sterilisierung mit Ethylenoxid (EO) und Rückgewinnung des EO gemäß dem Oberbegriff des geltenden Anspruchs 1.

Zur Sterilisation von medizinischen Geräten, auch verpackten, finden üblicherweise Alkylenoxide, insbesondere Ethylenoxid (EO), die zumeist mit inertisierenden Gasen wie beispielsweise Freon (Dichlordifluormetan) oder Kohlendioxid gemischt werden, Verwendung. Aus Kosten- und Umweltverträglichkeitsgründen werden die Gasgemische, d.h. das eigentliche Sterilisiergas und die inertisierenden weiteren Bestandteile, nach einem Sterilisiervorgang nicht einfach in die Umgebung abgelassen, sondern zurückgewonnen und wiederverwendet. Entsprechende Verfahren zur Sterilisierung und Rückgewinnung eines Sterilisiergases gehen aus der EP-A 0 130 319, der EP-A 0 326 985, der EP-A 0 417 592, der US-PS 3 549 312 sowie der US-PS 3 989 461 hervor.

Die bekannten Gasgemische aus EO und einem Freon sind auch bei höheren Drücken und einer Mischung mit Luft nicht explosibel. Die dabei verwendete Gaszusammensetzung von 12% EO und 88% Freon R12 läßt sich wegen des großen Dichteunterschiedes zwischen gasförmigem EO und gasförmigem R12 leicht auch in der gasförmigen Phase herstellen. Freone üben jedoch die bekannte schädliche Wirkung auf die Erdozonschicht aus und sollten deshalb bereits aus Gründen der Umweltverträglichkeit nicht mehr verwendet werden.

Das weiterhin oft benutzte Gasgemisch aus EO und Kohlendioxid weist den Nachteil einer vergleichsweise schwierigen EO-Rückgewinnung auf. Eine teilweise Rückgewinnung des EOs durch Kondensation ist zwar möglich, jedoch nur bis zu Dampfdrücken von etwa 5 mbar, was einer Temperatur von -78°C , dem Verfestigungspunkt von Kohlendioxid, entspricht. Es müßten daher größere Mengen EO entsorgt werden. Ferner ist es auch schwierig, aus dem wiedergewonnenen EO und Kohlendioxid in der Gasphase die richtige Gasmischung herzustellen, da EO und Kohlendioxid das gleiche Molekulargewicht besitzen. Eine Herstellung des Gemisches ist im allgemeinen nur in Chemiewerken bei Drücken über 5 bar möglich. Unter 5 bar geht Kohlendioxid direkt von der Gasphase in die feste Phase über.

Aufgrund dieser Schwierigkeiten wird bei den bestehenden Sterilisieranlagen, die das Gemisch EO/Kohlendioxid benutzen, auf eine EO-Rückgewinnung verzichtet.

Aus dem nicht vorveröffentlichten deutschen Patent Nr. 41 38 321 geht die kontinuierliche Spülung einer Sterilisierkammer mit Stickstoff hervor. Nach dem Sterilisieren, das etwa bei Umgebungsdruck stattfindet, erfolgt nach diesem Verfahren die Rückgewinnung des EO bei kontinuierlich sinkendem EO und entsprechend kontinuierlich steigendem Stickstoffanteil in der Sterilisierkammer. Für das Sterilisieren selbst wird eines der üblichen Gasgemische verwendet, wobei auch ein Gemisch aus EO und Stickstoff genannt wird.

Die Erfindung hat sich die Aufgabe gestellt, die mit den aus dem Stand der Technik bekannten Lösungen verbundenen Nachteile zu verringern und weitmöglichst zu vermeiden; insbesondere soll eine wirksame Sterilisierung mit EO und eine nahezu vollständige Rückgewinnung des EO möglich sein. Weiterhin soll von einer dem Inertisieren des EO dienenden Gasbeimischung keine Schädigung der Erdozonschicht ausgehen. Explosible Gasgemische sollen in einer Sterilisierkammer und in einem Sterilisiergas-Rückgewinnungskreislauf weitestgehend vermieden werden. Schließlich sollen bestehende EO/Kohlendioxid-Anlagen um den Aufbereitungsteil für das Gasgemisch in der Gasphase erweitert werden können.

Diese Aufgabe wird durch den Gegenstand des Anspruchs 1 gelöst.

Weitere vorteilhafte, nicht ganz selbstverständliche Ausgestaltungen der Erfindung werden durch die Unteransprüche offenbart.

Bei einem Verfahren zur sterilisierung mit Ethylenoxid (EO) und Rückgewinnung des EO werden die zu sterilisierenden Materialien in eine Sterilisierkammer verbracht und die Kammer zunächst auf einen Druck von etwa 100 mbar oder weniger evakuiert.

Die Sterilisierung findet erfindungsgemäß in einer gasförmigen Kammeratmosphäre bei einem Gasgemisch aus dem EO, Stickstoff und der nach der Evakuierung der Kammer verbliebenen Restluft statt, wobei der EO-Anteil vorteilhafterweise etwa 10-55% beträgt. Dazu wird die Kammer im Anschluß an die vorangegangene Evakuierung soweit bedruckt, daß die in der Kammer verbliebene Restluft weniger als etwa 10 - Vol.% der Kammeratmosphäre ausmacht. Nach der Einwirkung des Gasgemisches wird die Kammer unter Abzug des Gasgemisches wieder auf einen Druck von etwa 100 mbar oder weniger evakuiert und aus dem abgezogenen Gasgemisch das EO in einem Tieftemperaturkondensator zurückgewonnen. Danach wird die Kammer mindestens einmal mit Stickstoff wiederbedruckt und anschließend wieder auf einen Druck von etwa 100 mbar oder weniger evakuiert und vorteilhafterweise weiteres EO zurückgewonnen.

Die erfindungsgemäße Verwendung von Stickstoff als inertisierendem Gas hat den Vorteil, daß EO aus dem Kammergemisch durch einfache Kondensation in einem Tieftemperaturkondensator vom Stickstoff getrennt werden kann. Darüberhinaus ist Stickstoff ein sehr umweltverträgliches Gas. Während des gesamten Verfahrens bleibt der Restluftgehalt in der Kammer unterhalb von etwa 10%. Die Kammer wird nach der Evakuierung zum Sterilisieren vorteilhafterweise auf über etwa 1 bar Druck, besonders bevorzugt auf über etwa 1.3 bar bedruckt. Bei einem Druck von erfindungsgemäß etwa 2 bis 3 bar beträgt der Restluftgehalt in der Kammer höchstens noch etwa 5%. Das Gasgemisch gerät daher nicht in die Nähe der bei dem EO-Sterilisierverfahren gefürchteten Explosionsgrenze. Grundsätzlich kann der Kammerdruck bei erfindungsgemäßen Verfahrensführungen jedoch durchaus im Bereich von 0.5 bis 5 bar liegen. Durch einen oberhalb des Atmosphärendrucks liegenden Kammerdruck wird gleichzeitig einem Eindringen von Umgebungsluft in die Kammer vorgebeugt.

Zum Bedrucken der Kammer mit dem Gasgemisch wird das Gasgemisch vorteilhafterweise in seiner für die Sterilisierung gewünschten Zusammensetzung zugeführt. Gemäß einer weiteren bevorzugten Ausführungsform der Erfindung kann zur Erhöhung der Sicherheit die Kammer auch zunächst mit gasförmigem Stickstoff und erst anschließend mit dem EO in der gewünschten Menge bedruckt werden.

Nach dem Einwirken des Gasgemisches, d.h nach der Sterilisierung, wird die Kammer unter Abzug des Gasgemisches gleichzeitiger EO-Rückgewinnung in einem Tieftemperaturkondensator wieder auf einen Druck von etwa 100 mbar oder weniger evakuiert und danach die Kammer mindestens einmal mit reinem Stickstoff wieder auf einen Druck von bevorzugterweise etwa 1 bar gebracht. Nach diesem Schritt sind in dem Gasgemisch in der Kammer allenfalls noch 1% Restluftanteile vorhanden.

Die Rückgewinnung des EO erfolgt bevorzugterweise in einem Rückgewinnungskreislauf mit einem Tieftemperaturkondensator. Wird als Kühlmittel für die Tieftemperaturkondensation wiederum Stickstoff eingesetzt, so kann der bei der Kondensation verdampfte Kühlmittel-Stickstoff selbst wieder für nachfolgende Sterilisier- oder Spülzyklen eingesetzt werden.

An das zweite Auffüllen der Kammer können sich nach Bedarf weitere Spülzyklen mit Stickstoff oder auch Luft unter weiterer Rückgewinnung der EO-Reste anschließen. Zuvor wird die Kammer bevorzugterweise jeweils wiederum auf einen Druck von etwa 100 mbar, gegebenenfalls auch weniger, evakuiert. Vorteilhafterweise wird bei diesen Schritten jeweils EO zurückgewonnen.

Bei keinem der erfindungsgemäßen, definierten Evakuierungs- und Widerbedruckungsschritten gerät das Kammergemisch in die Nähe der Explosionsgrenze.

Besondere Bedeutung kommt neben der Zusammensetzung des der Kammer zugeführten Gemisches aus EO und Stickstoff, daß sich vorteilhafterweise auch erst in der Kammer ergeben kann, den erfindungsgemäß aufeinander folgenden Evakuierungs- und Bedruckungsschritten bzw. deren Druckniveaus zu.

Zurückgewonnen wird reines EO in flüssiger Form, das zur Wiederverwendung verdampft wird.

Im Nachfolgenden wird eine besonders bevorzugte Verfahrensführung nach der Erfindung beschrieben. Dabei findet ein Gasgemisch von etwa jeweils 50% EO und Stickstoff Verwendung.

Vor dem ersten Verfahrensschritt nach der Erfindung befinden sich die zu sterlisierenden Materialien in der auf einen Druck von etwa 100 mbar evakuierten Sterilisierkammer. Zu diesem Zeitpunkt besteht das Kammergemisch aus 100% Luft.

Im zweiten Schritt wird eine Gasmischung von 50% EO und 50% Stickstoff in die Kammer geleitet und die Kammer auf einen Druck von etwa 2 bar gebracht. Das Kammergemisch besteht anschließend entsprechend aus jeweils 47,5% EO und Stickstoff sowie 5% Luft.

Im dritten Schritt wird die Kammer wieder auf einen Druck von etwa 100 mbar evakuiert. Die prozentuale Zusammensetzung des Kammergemisches bleibt dabei gegenüber dem vorhergehenden Schritt identisch.

Im vierten Schritt wird die Kammer mit reinem Stickstoff wieder auf einen Druck von etwa 1 bar bedruckt, so daß das Kammergemisch am Ende dieses Verfahrensschritts eine Zusammensetzung von 4,75% EO, 94,75% Stickstoff und 0,5% Restluft aufweist.

Beim anschließenden dritten Evakuierungsschritt wird die Kammer wiederum auf einen Druck von etwa 100 mbar ohne Änderung der Gemischzusammensetzung in der Kammer gebracht.

Durch weitere Spülungen der Kammer kann dann der EO-Gehalt des Kammergemisches nahezu auf 0% reduziert werden.

Die beschriebene Entwicklung der Zusammensetzung des Kammergemisches wird in der nachfolgenden Tabelle zusammen mit dem Kammerdruck nochmals zusammenfassend dargestellt:

| | % EO | % N2 | % Luft | Kammerdruck |
|---|---|---|---|---|
| Schritt 1: | 0 | 0 | 100 | 100 mbar |
| Schritt 2: | 47,5 | 47,5 | 5 | 2 bar |
| Schritt 3: | 47,5 | 47,5 | 5 | 100 mbar |
| Schritt 4: | 4,75 | 94,75 | 0,5 | 1 bar |
| Schritt 5: | 4,75 | 94,75 | 0,5 | 100 mbar |
| Schritt 6: | weiter abnehmend | | | |

## Patentansprüche

1. Verfahren zur Sterilisierung mit Ethylenoxid (EO) und Rückgewinnung des EO, bei dem die zu sterilisierenden Materialien in eine Sterilisierkammer verbracht und die Kammer zunächst auf einen Druck von 100 mbar oder weniger evakuiert wird,
**dadurch gekennzeichnet,** daß
a) anschließend die Kammer mit einem Gasgemisch aus dem EO und Stickstoff soweit bedruckt wird, daß die in der Kammer verbliebene Restluft wenigger als etwa 10 Vol.% der Kammeratmosphäre ausmacht,
b) die Kammer nach Einwirkung des Gasgemisches unter Abzug des Gasgemisches wieder auf einem Druck von etwa 100 mbar oder weniger evakuiert und aus dem abgezogenen Gasgemisch das EO in einem Tieftemperaturkondensator zurückgewonnen,
c) danach die Kammer mindestens einmal mit Stickstoff bedruckt und
d) anschließend wieder auf einen Druck von etwa 100 mbar oder weniger evakuiert wird.

2. Verfahren nach Anspruch 1, dadurch gekennzeichnet, daß das der Kammer zugeführte Gasgemisch etwa 10 bis 55 % EO enthält.

3. Verfahren nach Anspurch 1 oder 2, dadurch gekennzeichnet, daß in 1a) die Kammer mit dem Gasgemisch auf mindestens etwa 1,3 bar, insbesondere auf etwa 2 bis 3 bar, bedruckt wird.

4. Verfahren nach einem der Ansprüche 1 bis 3, dadurch gekennzeichnet, daß in 1a) die Kammer zunächst mit Stickstoff und danach mit dem EO bedruckt wird.
